(19) **Europäisches Patentamt**
**European Patent Office**
**Office européen des brevets**

(11) **EP 4 141 879 B1**

(12) **EUROPEAN PATENT SPECIFICATION**

(45) Date of publication and mention
of the grant of the patent:
**29.04.2026 Bulletin 2026/18**

(21) Application number: **22191622.4**

(22) Date of filing: **23.08.2022**

(51) International Patent Classification (IPC):
**G16H 20/17** (2018.01)     **G16H 40/63** (2018.01)
**G16H 50/20** (2018.01)     **G16H 50/30** (2018.01)

(52) Cooperative Patent Classification (CPC):
**G16H 20/17; G16H 50/20; G16H 50/30**

(54) **ADJUSTMENT OF INSULIN-TO-CARBOHYDRATE RATIO, CORRECTION FACTOR, INSULIN DOSAGE AND TIMING OF INSULIN DELIVERY BY A MEDICAMENT DELIVERY DEVICE**

EINSTELLUNG DES INSULIN-KOHLENHYDRAT-VERHÄLTNISSES, DES KORREKTURFAKTORS, DER INSULINDOSIERUNG UND DES ZEITPUNKTS DER INSULINABGABE DURCH EINE MEDIKAMENTENABGABEVORRICHTUNG

RÉGLAGE DU RAPPORT INSULINE/GLUCIDE, FACTEUR DE CORRECTION, DOSAGE D'INSULINE ET SYNCHRONISATION D'ADMINISTRATION D'INSULINE À L'AIDE D'UN DISPOSITIF D'ADMINISTRATION DE MÉDICAMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.08.2021 US 202163236264 P**

(43) Date of publication of application:
**01.03.2023 Bulletin 2023/09**

(73) Proprietor: **Insulet Corporation**
**Acton, MA 01720 (US)**

(72) Inventors:
• **LEE, Joon Bok**
**Acton (US)**

• **ZADE, Ashutosh**
**San Diego (US)**
• **ZHENG, Yibin**
**Hartland (US)**
• **O'CONNOR, Jason**
**Acton (US)**

(74) Representative: **Peterreins Schley**
**Patent- und Rechtsanwälte PartG mbB**
**Hermann-Sack-Straße 3**
**80331 München (DE)**

(56) References cited:
**WO-A1-2021/163324     US-A1- 2021 170 104**
**US-B2- 9 233 204**

**Description**

**BACKGROUND**

A patient with Type 1 diabetes mellitus may manually receive insulin by injecting shots of insulin. In order to determine the appropriate dosages of insulin to deliver, the patient may rely on values, such as the insulin-to-carbohydrate ratio (ICR) and the correction factor. The ICR specifies how many units of insulin are needed to compensate for 1 gram of ingested carbohydrates. The correction factor refers to how much 1 unit of insulin will lower the glucose concentration of a person in a fasting or pre-meal state over a period of 2-4 hours. The ICR and the correction factor are customized to the patient.

If the patient becomes a user of a conventional insulin delivery device, the conventional insulin delivery device may make an estimate of an appropriate ICR and correction factor for the patient. However, these estimates may not prove to be very accurate and the delivery of insulin to the patient may be not very well tailored to the patient.

With some conventional insulin delivery devices, the user may request delivery of a bolus of insulin. The onus is on the user to decide the dosage of the bolus and when to deliver the dosage. The user must enter either the dosage amount or a best guess of the number of carbohydrates to be consumed. In some circumstances, the user may cause the delivery of the bolus too soon and run the risk of hypoglycemia. In addition, the user may select a bolus dosage that does not match their true needs or, alternatively, get a bolus dosage that does not match their true needs by inaccurately estimating the carbohydrates ingested. Moreover, the timing of delivery of the dosage may be up to the user. The user may have to make an estimate of when the user will ingest a meal. In some instances, the user underestimates the time before meal ingestion. In such cases, the insulin bolus that has been delivered to compensate for meal ingestion may drive down the user's glucose concentration before the glucose resulting from digestion of the meal is digested. This and other scenarios can result in hypoglycemia for the user.

**SUMMARY**

In accordance with an inventive aspect, a method is performed by a processor for controlling a medicament delivery device. In the method, data regarding medicament delivery and glucose levels of a user is gathered during operation of the medicament delivery device. The gathered data is analyzed with the processor to identify factors indicating that an insulin to carbohydrate ratio for the user or a correction factor for the user should be increased. The gathered data also is analyzed with the processor to identify factors indicating that the insulin to carbohydrate ratio for the user or the correction factor should be decreased. The identified factors are weighed with the processor to decide whether to increase or decrease the insulin to carbohydrates ratio or the correction factor. The insulin to carbohydrate ratio or the correction factor for the user are increased or decreased based on the weighing of the identified factors.

The identified factors indicating that an insulin to carbohydrate ratio for the user or a correction factor for the user should be increased may include at least one of the user experiencing hyperglycemia in a time interval, the user delivered less than a threshold number of insulin boluses in a time period, the user experienced a glucose concentration above an elevated threshold for a duration, or non-bolus medicament delivery to the user exceeds a percentage threshold. The identified factors indicating that the insulin to carbohydrate ratio for the user or the correction factor should be decreased may include at least one of the user experiencing hypoglycemia in a time interval, the user delivered more than a threshold number of insulin boluses in a time period, the user experienced a glucose concentration below a depressed threshold for a duration, or bolus medicament delivery to the user exceeds a percentage threshold, variability among glucose concentration readings for the user is greater than a variability threshold, or the user activates a mode to protect against hypoglycemia more often than a frequency threshold. Weighing the identified factors may include assigning weights to the identified factors. Some of the weights may be positive and some of the weights may be negative. The method may include summing the weights to produce a sum and comparing the sum to one or more weight thresholds to decide whether to increase or decrease the insulin to carbohydrate ratio or the correction factor. The increasing or decreasing the insulin to carbohydrate ratio or the correction factor may include increasing both the insulin to carbohydrate ratio and the correction factor. The increasing or decreasing the insulin to carbohydrate ratio or the correction factor as decided may comprise increasing or decreasing both the carbohydrate ratio and the correction factor. In an embodiment not covered by the present invention, the method may include delivering insulin to the user with the medicament delivery device with a dosage based on the increased or decreased insulin to carbohydrate ratio or the increased or decreased correction factor.

In accordance with another inventive aspect, a method is performed by a processor for controlling a medicament delivery device. Per the method, a sum of deviations is determined by the processor. The sum of deviations is a sum of deviations between glucose concentration values for a user over a time interval following a delivery of a first insulin bolus dosage that was determined by the processor and a larger of a glucose concentration value at a time of delivering the first insulin bolus dosage or a hyperglycemia threshold expressed as a glucose concentration value. A next insulin bolus dosage calculation is adjusted to increase the next insulin bolus dosage relative to the first insulin bolus dosage with the processor based on the determined sum of deviations.

The method may include establishing an upper limit based on how much the next insulin bolus dosage may be adjusted relative to the first insulin bolus dosage. The method further may include establishing a lower limit based on how much the next insulin bolus dosage may be adjusted relative to the first insulin bolus dosage. In an embodiment not covered by the present invention, the method also may include delivering the next insulin bolus dosage to the user with the medicament delivery device. The method additionally may include detecting with the processor when an increase in glucose concentration for the user begins after the delivering of the first insulin bolus dosage, and if the increase in glucose concentration is detected to begin after a specified time interval after the delivering of the first insulin bolus dosage, delivering a first portion of the next insulin bolus with the medicament delivery device and delaying the delivery of a remaining portion of the next insulin bolus dosage by the medicament delivery device a determined period of time.

In accordance with a further inventive aspect, a method is performed by a processor for controlling a medicament delivery device. The method includes determining with the processor how much time elapses after delivery of an insulin bolus before glucose concentration of a user increases. The method further includes, with the processor, comparing the determined time that elapsed after delivery of the insulin bolus before glucose concentration of the user increases relative to a threshold amount of time. If the comparing indicates that the determined time is greater than the threshold amount of time, delivery by the medicament delivery device to the user of at least a portion of a next insulin bolus requested by the user is delayed.

Delivery of a percentage Y of the insulin bolus dosage by the medicament delivery device to the user may be delayed, where Y is a tunable parameter. Initially, 100% - Y% of the insulin bolus may be delivered by the medicament delivery device to the user. The delaying may comprise delaying the delivery by Z number of cycles, where Z is a tunable parameter. Y may be greater than 15%, and Z may be 6 cycles, for example. Delivery by the medicament delivery device to the user of the next insulin bolus requested by the user may not be delayed if the comparing indicates that the determined time is not greater than the threshold amount of time.

US 2021/170104 A1 discloses a device for controlling insulin delivery by optimizing glycaemic control based on a "suspend status" of the pump.

## BRIEF DESCRIPTION OF THE DRAWINGS

Figure 1 depicts an illustrative medicament delivery system that is suitable for exemplary embodiments.

Figure 2 depicts a flowchart of illustrative steps that may be performed in exemplary embodiments to adjust ICR and/or correction factor values.

Figure 3A depicts a table of illustrative factors that weigh towards increasing ICR and/or correction factor.

Figure 3B depicts a table of illustrative factors that weigh towards decreasing ICR and/or correction factor.

Figure 3C depicts illustrative actions that may be taken responsive to the summed weights in exemplary embodiments.

Figure 4A depicts an illustrative plot of glucose concentration values.

Figure 4B depicts an illustrative plot of medicament delivery amounts over time.

Figure 5 depicts a flowchart of illustrative steps that may be performed in exemplary embodiments to adjust insulin bolus dosages and timing of delivery of insulin boluses requested by a user.

Figure 6 depicts a flowchart of illustrative steps that may be performed in exemplary embodiments to automatically adjust user requested insulin boluses.

Figure 7 depicts a flowchart of illustrative steps that may be performed in exemplary embodiments to calculate a bolus adjustment factor.

Figure 8 depicts a flowchart of illustrative steps that may be performed in exemplary embodiments to adjust the timing of delivery of an insulin bolus.

Figure 9A depicts an illustrative plot of glucose concentration values.

Figure 9B depicts an illustrative plot of insulin delivery amounts over time.

**DETAILED DESCRIPTION**

The exemplary embodiments may automatically adjust the ICR and the correction factor for a user of a medicament delivery device. The automatic adjustments may tailor the values to the user's actual insulin needs. The ICR and the correction factor may be used by an automatic insulin delivery (AID) control algorithm of the medicament delivery device in determining an insulin dosage for the user. The exemplary embodiments may look to various factors to determine how to adjust the ICR and the correction factor. These factors may include factors for increasing the ICR and/or the correction factor, and factors for decreasing the ICR and/or the correction factor. The different varieties of factors may counterbalance each other. As such, the exemplary embodiments may weigh the totality of the different variety of factors to determine whether to increase or decrease the ICR and/or at the correction factor and by how much to increase or decrease the ICR and/or the correction factor. In some instances where the factors counterbalance each other, it may be determined that no adjustments need to be made.

The exemplary embodiments also may make automatic adjustments of user-requested insulin boluses both in terms of requested dosages and timing of deliveries of the insulin boluses. Specifically, the exemplary embodiments may determine whether the magnitude of the insulin boluses requested by the user are consistent with the post-bolus glucose concentration control outcomes for past user-requested insulin boluses. The post-bolus glucose concentration control outcomes may indicate that the user is under-estimating the dosage in some instances. In some exemplary embodiments, a determination may be made regarding whether the deviations of glucose concentrations relative to the glucose concentration at the time of administering a bolus are excessive relative to a tolerance level. If it is determined that the deviations are excessive, an adjustment factor may be applied to subsequent insulin bolus dosages to increase the dosage is to avoid excessive positive deviations.

The exemplary embodiments may also scrutinize the timing of the delivery of manual insulin boluses requested by the user. In particular, the exemplary embodiments may look to see if the insulin boluses are delivered too early (i.e., more than 15 minutes before meal ingestion). Such early delivery of insulin boluses increases the risk of the user experiencing hypoglycemia as the insulin will decrease glucose concentration prior to an offset increase due to meal ingestion. The exemplary embodiments may identify early delivery of insulin boluses by looking for when the glucose concentration of the user begins to increase. If the increase is less than a threshold period, such as 30 minutes since the insulin bolus delivery, the user likely delivered an insulin bolus no more than a threshold period of time prior to meal ingestion. This is not especially problematic. However, if the glucose concentration increased after the threshold period, it is potentially problematic as the delay in the increase in glucose concentration is indicative of a delay in meal ingestion, and as a result, the user is running a risk of hypoglycemia. In such instances, the exemplary embodiments may deliver a percentage of the insulin bolus dosage initially and deliver the remaining percentage after a delay to reduce the risk of hypoglycemia for the user. Thus, the amount of insulin delivered initially is less to reduce the risk of hypoglycemia. The remainder of the insulin is delivered after a delay where an increase in glucose concentration from meal ingestion is more likely.

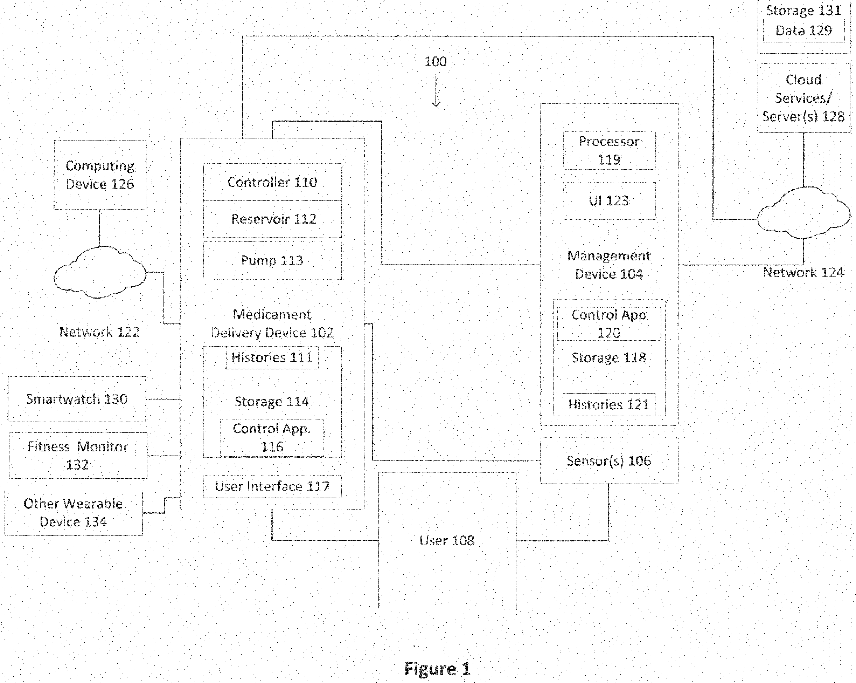

Figure 1 depicts an illustrative medicament delivery system 100 that is suitable for delivering a medicament to a user 108 in accordance with exemplary embodiments. The medicament delivery system 100 includes a medicament delivery device 102. The medicament delivery device 102 may be a wearable device that is worn on the body of the user 108 or carried by the user and having an infusion site. The medicament delivery device 102 may be directly coupled to a user (e.g., directly attached to a body part and/or skin of the user 108 via an adhesive or the like) or carried by the user (e.g., on a belt or in a pocket) with tubing connecting the medicament delivery device 102 to an infusion site where the medicament is injected. In a preferred embodiment, a surface of the medicament delivery device 102 may include an adhesive to facilitate attachment to the user 108.

The medicament delivery device 102 may include a controller 110. The controller 110 may, for example, include a microprocessor, a logic circuit, a field programmable gate array (FPGA), an application specific integrated circuit (ASIC) or a microcontroller. The controller 110 may maintain a date and time as well as other functions (e.g., calculations or the like). The controller 110 may be operable to execute a control application 116 encoded in computer programming instructions stored in the storage 114 that enables the controller 110 to direct operation of the medicament delivery device 102.

The control application 116 may control delivery of a medicament to the user 108 per a control approach like that described herein. The storage 114 may hold histories 111 for a user, such as a history of basal deliveries, a history of bolus deliveries, and/or other histories, such as a meal event history, exercise event history and/or the like. In addition, the controller 110 may be operable to receive data or information. The storage 114 may include both primary memory and secondary memory. The storage may include random access memory (RAM), read only memory (ROM), optical storage, magnetic storage, removable storage media, solid state storage or the like.

The medicament delivery device 102 may include one or more housings for housing its various components including a pump 113, a power source, and a reservoir 112 for storing a medicament for delivery to the user 108 as warranted. A fluid path to the user 108 may be provided, and the medicament delivery device 102 may expel the medicament from the reservoir 112 to deliver the medicament to the user 108 using the pump 113 via the fluid path. The fluid path may, for example, include tubing coupling the medicament delivery device 102 to the user 108 (e.g., tubing coupling a cannula to the reservoir 112), and may include tubing to a separate infusion site.

There may be one or more communications links with one or more devices physically separated from the medicament delivery device 102 including, for example, a management device 104 of the user and/or a caregiver of the user and/or a sensor 106. The communication links may include any wired or wireless communication links operating according to any known communications protocol or standard, such as Bluetooth®, Wi-Fi, a near-field communication standard, a cellular standard, or any other wireless protocol. The medicament delivery device 102 may also include a user interface 117, such as an integrated display device for displaying information to the user 108 and in some embodiments, receiving information from the user 108. The user interface 117 may include a touchscreen and/or one or more input devices, such as buttons, knobs, or a keyboard.

The medicament delivery device 102 may interface with a network 122. The network 122 may include a local area network (LAN), a wide area network (WAN) or a combination therein. A computing device 126 may be interfaced with the network, and the computing device may communicate with the medicament delivery device 102.

The medicament delivery system 100 may include sensors 106 for sensing the levels of one or more analytes. The sensors 106 may be coupled to the user 108 by, for example, adhesive or the like and may provide information or data on one or more medical conditions and/or physical attributes of the user 108. The sensors 106 may be physically separate from the medicament delivery device 102 or may be an integrated component thereof.

The medicament delivery system 100 may or may not also include a management device 104. In some embodiments, no management device is not needed as medicament delivery device 102 may manage itself. The management device 104 may be a special purpose device, such as a dedicated personal diabetes manager (PDM) device. The management device 104 may be a programmed general-purpose device, such as any portable electronic device including, for example, a dedicated controller, such as processor, a micro-controller, or the like. The management device 104 may be used to program or adjust operation of the medicament delivery device 102 and/or the sensors 106. The management device 104 may be any portable electronic device including, for example, a dedicated device, a smartphone, a smartwatch or a tablet. In the depicted example, the management device 104 may include a processor 119 and a storage 118. The processor 119 may execute processes to manage a user's glucose levels and to control the delivery of the medicament to the user 108. The medicament delivery device 102 may provide data from the sensors 106 and other data to the management device 104. The data may be stored in the storage 118. The processor 119 may also be operable to execute programming code stored in the storage 118. For example, the storage may be operable to store one or more control applications 120 for execution by the processor 119. The one or more control applications 120 may be responsible for controlling the medicament delivery device 102, such as by controlling the AID delivery of insulin to the user 108. The storage 118 may store the one or more control applications 120, histories 121 like those described above for the medicament delivery device 102, and other data and/or programs.

The management device 104 may include a user interface (UI) 123 for communicating with the user 108. The user interface 123 may include a display, such as a touchscreen, for displaying information. The touchscreen may also be used to receive input when it is a touch screen. The user interface 123 may also include input elements, such as a keyboard, button, knobs, or the like.

The management device 104 may interface with a network 124, such as a LAN or WAN or combination of such networks. The management device 104 may communicate over network 124 with one or more servers or cloud services 128. Data, such as sensor values, may be sent, in some embodiments, for storage and processing from the medicament delivery device 102 directly to the cloud services/server(s) 128 or instead from the management device 104 to the cloud services/server(s) 128. The cloud services/server(s) 128 may provide output from the model 115 as needed to the management device 104 and/or medicament delivery device 102 during operation.

Other devices, like smartwatch 130, fitness monitor 132 and wearable device 134 may be part of the delivery system 100. These devices may communicate with the medicament delivery device 102 to receive information and/or issue commands to the medicament delivery device 102. These devices 130, 132 and 134 may execute computer programming instructions to perform some of the control functions otherwise performed by controller 110 or processor 119, such as via control applications 116 and 120. These devices 130, 132 and 134 may include displays for displaying information. The displays may show a user interface for providing input by the user, such as to request a change or pause in dosage or to request, initiate, or confirm delivery of a bolus of a medicament, or for displaying output, such as a change in dosage (e.g., of a basal delivery amount) as determined by controller 110 or management device 104. These devices 130, 132 and 134 may also have wireless communication connections with the sensor 106 to directly receive analyte measurement data.

A wide variety of medicaments may be delivered by the medicament delivery device 102. The medicament may be insulin for treating diabetes. The medicament may be glucagon for raising a user's glucose level. The medicament may also be a glucagon-like peptide (GLP)-1 receptor agonists for lowering glucose or slowing gastric emptying, thereby delaying spikes in glucose after a meal.

The adjustments made by the exemplary embodiments may be under the control of the control application 116 of the medicament delivery device 102 or the control application 120 of the management device 104. In some embodiments, the adjustments may be made by the cloud services or servers 128, the computing device 126 or by the other enumerated devices, including smartwatch 130, fitness monitor 132 or another wearable device 134.

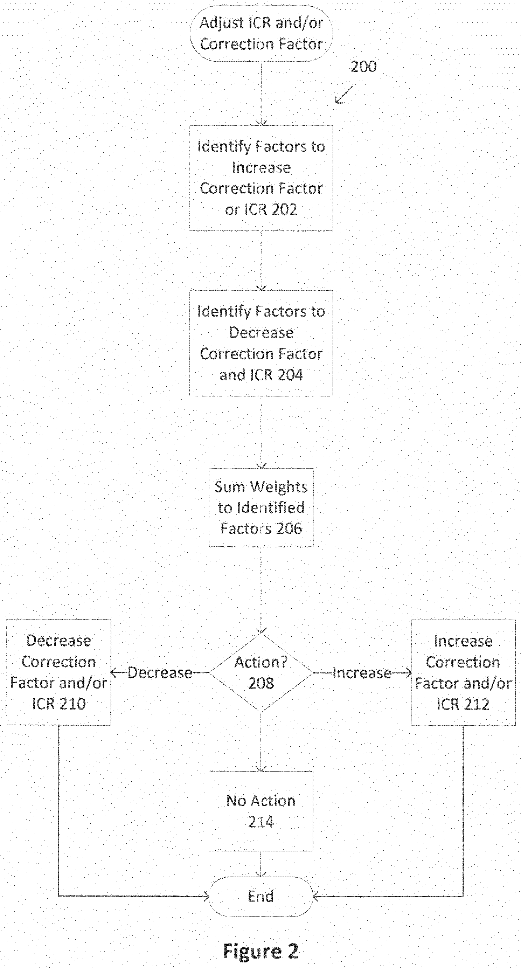

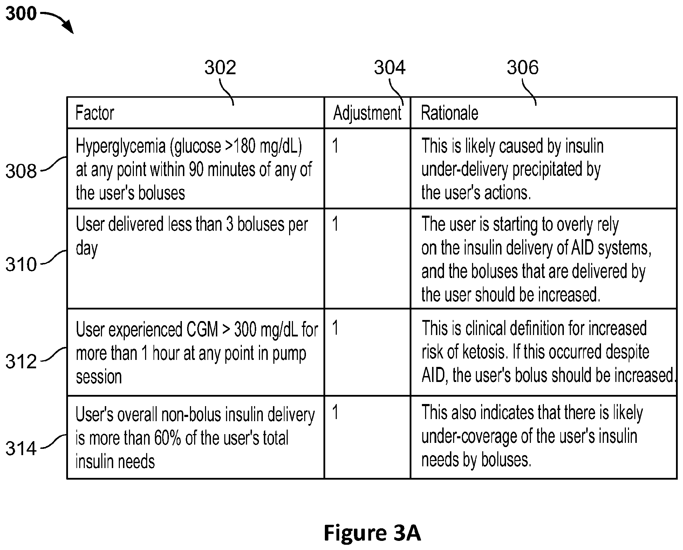

Figure 2 depicts a flowchart 200 of illustrative steps that may be performed in an exemplary embodiment to adjust the ICR and/or the correction factor responsive to different varieties of factors. Initially, at 202, the exemplary embodiments identify factors for increasing the correction factor and/or the ICR of the user. Figure 3A depicts a table 300 of illustrative factors that may be identified and count towards increasing the correction factor and/or the ICR of the user. It should be appreciated that other factors may be used. The table 300 includes columns 302, 304 and 306. Column 302 holds a description of the nature of the factor. Column 304 identifies the magnitude of the adjustment weight associated with the factor. Column 306 provides a brief explanation of the rationale for the factor. Each row 308, 310, 312 and 314 is associated with a respective factor.

The factor 302 of row 308 is whether the use has experienced hyperglycemia (i.e., a glucose concentration reading for the user of over 180 mg/dL) at any point within 90 minutes after delivery of an insulin bolus. An adjustment weight 304 of +1 is associated with this factor. The rationale 306 for this factor is that any incidence of hyperglycemia is likely caused by under-delivery of insulin due to user action.

The factor 302 of row 310 is whether the user delivered less than three boluses per day. This factor is associated with an adjustment weight 304 of +1. The rationale 306 for this factor is that if the user delivered less than three boluses per day, the user is relying too greatly on the automated insulin delivery (e.g., basal insulin delivery by the medicament delivery device 102) for their insulin needs and that the number of boluses per day should be increased.

The factor 302 of row 312 is whether the user experienced a glucose concentration reading of greater than 300 mg/dL for more than an hour. This factor is associated with an adjustment weight 304 of +1. The rationale 306 for this factor is that it corresponds with the clinical definition for increased risk of ketosis and hence, the number boluses should be increased.

The factor 302 of row 314 is that the user's overall non-bolus insulin delivery is more than 60% of the user's total insulin needs rather than a more ideal 50% of the total insulin needs. This factor is associated with an adjustment weight 304 of +1. The rationale 306 for this factor is that it is indicative of under-delivery of boluses.

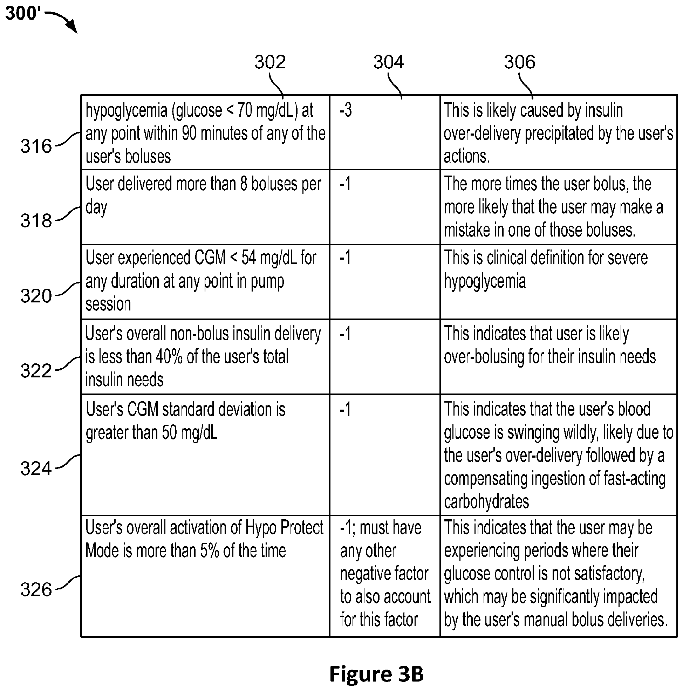

With reference to Figure 2, at step 202, the exemplary embodiments process data stored in the histories 111 to identify whether any of the factors are present or not. At 204, factors to decrease the ICR and/or correction factor are identified. Figure 3B depicts a table 300' of illustrative factors to decrease the ICR and/or correction factor. The table 300' includes rows 316, 318, 320, 322, 324 and 326, where each row is associated with a respective factor. Each row includes an identification of the factor 302, and adjustment value 304 and a rationale 306.

The factor 302 of row 316 is whether the user has experienced hypoglycemia (i.e., glucose concentration of < 70 mg/dL) within X minutes (e.g., 90 minutes) of a user's bolus. The adjustment weight 304 for such an occurrence is -3. This factor is weighed more heavily than other factors. The rationale 306 for this factor 302 is that the occurrence of hypoglycemia is likely caused by insulin over-delivery due to the actions of the user.

The factor 302 of row 318 is that the user delivered more than eight boluses per day. An adjustment weight 304 of -1 is associated with this factor. The rationale 306 for the factor is that the user is more likely to make a mistake with bolus dosing the more times the user delivers boluses in a day.

The factor of row 320 is that the user experienced a glucose concentration level of less than 54 mg/dL for any duration. This factor is associated with an adjustment weight 304 of -1. The rationale 306 for this factor is that the factor corresponds with a clinical definition for severe hypoglycemia.

The factor 302 of row 322 is that the user delivers less than 40% of the user's total insulin needs by non-bolus delivery. This factor is associated with an adjustment weight 304 of -1. The rationale 306 for this factor is that the factor is indicative of likely excessive boluses by the user.

The factor 302 of row 324 is that the user's standard deviation in glucose concentration values exceeds 50 mg/dL. This factor is associated with an adjustment weight 304 of -1. The rationale for this factor is that this factor indicates that the user's glucose is swinging excessively, which may likely be due to over-delivery followed by a compensating ingestion of fast-acting carbohydrates.

The factor 302 of row 326 is that the user activates a hypoglycemia protection mode, for example Hypo Protect® mode, more than 5% of the time. This is a mode that seeks to protect the user from experiencing hypoglycemia. Typically, in the Hypo Protect mode, insulin dosages are reduced and/or insulin deliveries are temporarily halted. This factor is associated with an adjustment weight of -1, but there must be at least one other negative factor to include this factor in the overall calculus of weighted factors. The rationale 306 for this factor is that the factor indicates the user may be experiencing periods of unsatisfactory glucose control and that this may be the result of manual insulin bolus deliveries.

With reference to Figure 2, after the factors have been identified for the user in 202 and 204, the weights for the identified factors are summed at 206. In other words, the factors that apply are identified in steps 202 and 204, and the adjustment weights for the identified factors are summed. For example, if there are two factors that have an adjustment weight of +1 and another factor with adjustment weight of -3, the sum of the weights is -1. Based upon this sum, at 208, a determination is made whether to take any action or not. This determination is based upon whether the sum is above an upper threshold, below a lower threshold, or between the upper threshold and the lower threshold.

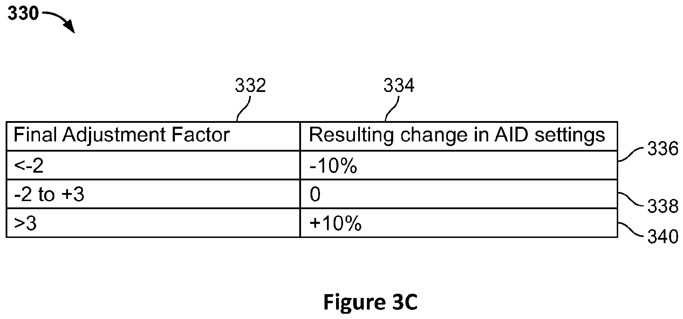

Figure 3C shows an illustrative table 330 indicating what action, if any, is to be taken based upon the calculated sum. The table 330 includes rows 336, 338 and 340. Each row 336, 338 and 340 is associated with a particular range of values for the sum. If the sum (i.e., final adjustment factor 332) is less than -2, the action 334 is that the ICR and/or correction factor are decreased by 10% at 210. If the sum 332 is in the range between -2 and +3, no action is taken at 214. If the sum 332 is greater than +3 the action 334 is that the ICR and/or correction factor are increased by 10% at 212. It should be appreciated that the specified rules are illustrative and that other rules may be applied.

The steps of the flowchart 200 of Figure 2 may be performed by a rules-based system. The system may be realized by fuzzy logic or by an artificial intelligence agent. Alternatively, the intelligence for performing the steps of the flowchart 200 may be realized in other types of software running on the controller 110 or processor 119.

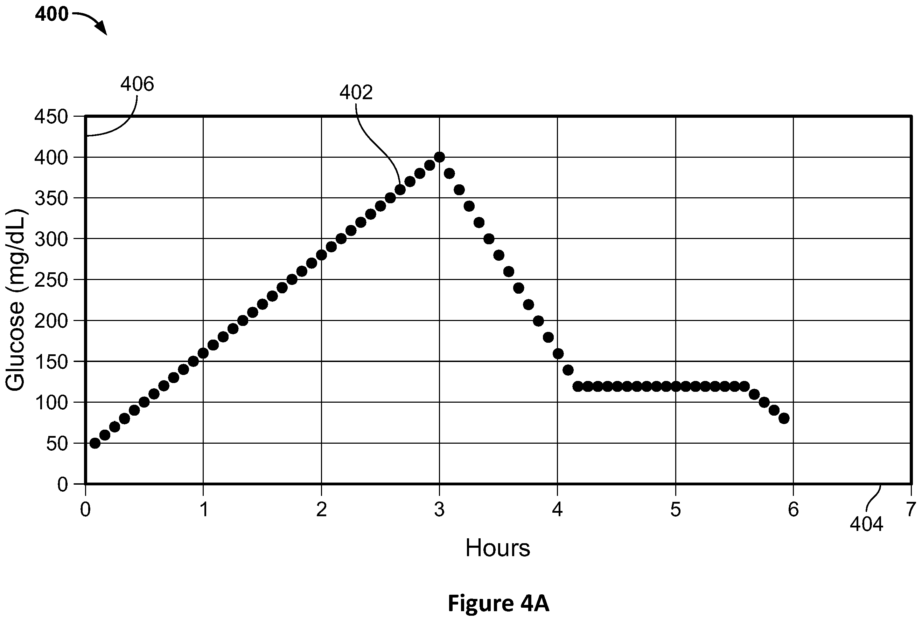

Figure 4A depicts a plot 400 of illustrative glucose values for a user. The X axis 404 is time, and the y-axis 406 is glucose concentration expressed in units of mg/dL. The curve 402 shows discrete values of the glucose concentration over time. As can be seen in Figure 4A, the glucose concentration of the user steadily increases from time 0 to 3 hours. The glucose concentration then decreases steadily between 3 hours to slightly after 4 hours.

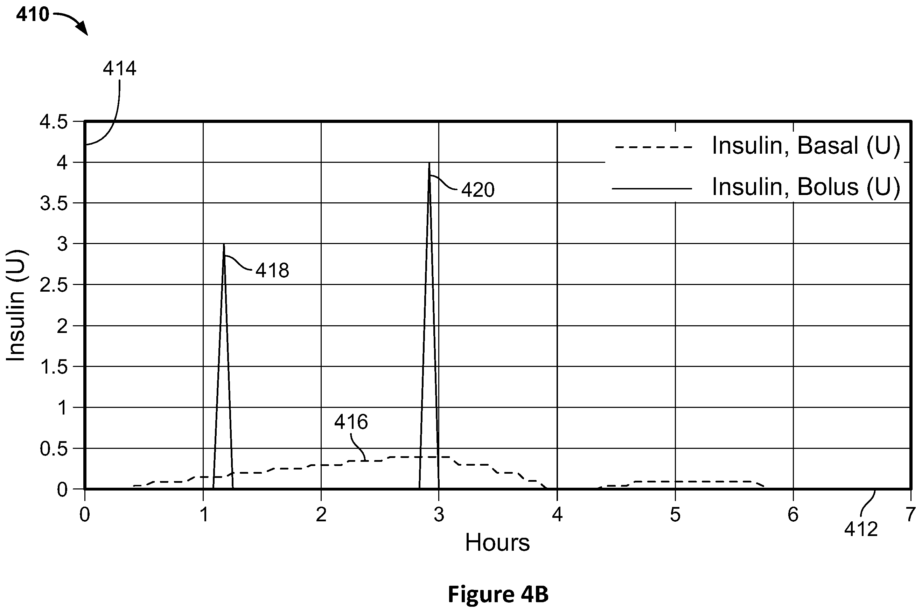

Figure 4B depicts the corresponding insulin deliveries over the same time period shown in Figure 4A. the X-axis 404 is time in hours, and the Y-axis 414 is insulin in units. Curve 416 corresponds to basal insulin delivery by the medicament delivery device. Peaks 418 and 420 correspond to insulin bolus deliveries. The bolus associated with insulin peak 420 causes in large part the decrease in glucose concentration shown in Figure 4A between hours 3 and 4. The halted delivery of basal insulin and the subsequent low level of basal insulin delivery between hours 4 and 6 results in the plateau and subsequent slight decline in glucose concentration of the user shown between hours 4 and 6 in Figure 4A.

Peak 420 is larger than peak 418. The increase may be due to the exemplary embodiments increasing the magnitude of the ICR and/or the correction factor due to the extended positive deviations depicted in Figure 4A between hours 1 and 3. As can be seen in Figure 4A, the increased bolus size helps to reduce the positive deviations.

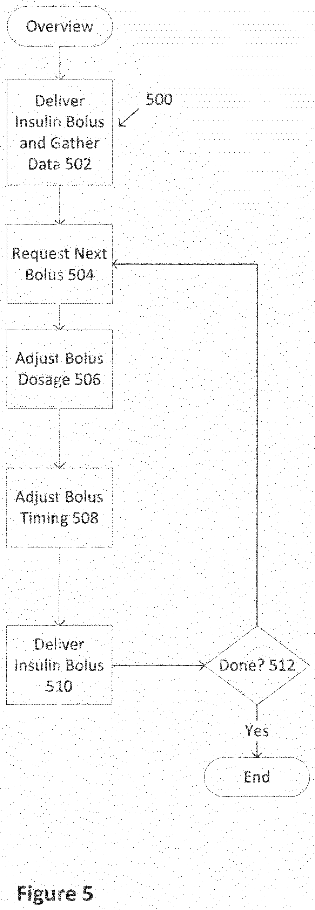

As was mentioned above, the exemplary embodiments may adjust user-initiated bolus deliveries in both timing and dosage amounts. These adjustments may be done automatically by the control application 116 or the control application 120 responsive to positive glucose concentration deviations. Figure 5 depicts a flowchart 500 of illustrative steps that may be performed in exemplary embodiments in order to make such automatic adjustments. At 502, the user requests an insulin bolus to be delivered, and subsequently, data is gathered. As will be explained in more detail below, the data is gathered to determine whether adjustments need to be made and to determine the nature of the adjustments, if warranted. The data may include glucose concentration values as well as when the last insulin bolus was delivered. In some exemplary embodiments, the gathered data may include additional types of data. At 504, the user requests a next insulin bolus to be delivered. At 506, the bolus dosage is adjusted if warranted. At 508, the timing of the delivery of the bolus is adjusted if warranted. At 510, the insulin bolus is delivered with any dosage adjustments and timing adjustments. At 512, the system checks whether it is done. If so, the processing stops. If not, the process repeats at 504 when the next bolus is requested. Thus, the adjustments may be made on a bolus-by-bolus-basis. The modifying of the ICR and/or correction factor may also affect the timing and dosage of basal deliveries as determined by the control application 116 or 120.

As was mentioned above, adjustments in the dosage of the user-requested insulin bolus may be made based on positive glucose concentration deviations following a delivery of a previous insulin bolus. The presence of the positive glucose concentration deviations may be indicative that the user is underestimating the proper insulin bolus dosage. The exemplary embodiments may identify that the user is underestimating the proper insulin bolus dosage based on the positive deviations of glucose concentration after delivery of an insulin bolus and may adjust the next requested insulin bolus dosage by an adjustment factor that increases the next insulin bolus dosage.

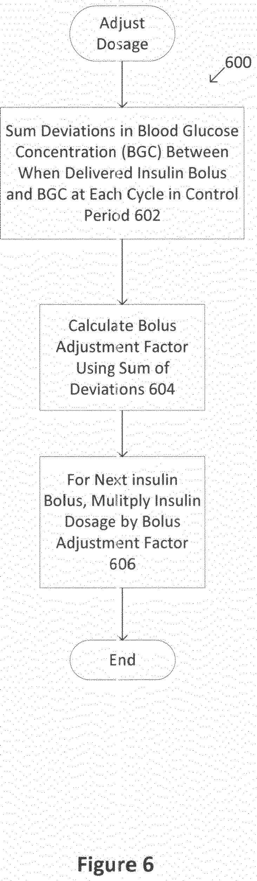

Figure 6 depicts a flowchart 600 of illustrative steps that may be performed in exemplary embodiments to adjust the dosage of the user requested insulin bolus based on positive glucose concentration deviations. At 602, the deviations of glucose concentration between when the insulin bolus was delivered at each cycle in a control period are summed. In the exemplary embodiments, the deviations of glucose concentration between when the insulin bolus was delivered at each cycle in the control period may be calculated as follows:

$$Q_b = \sum_{i=1}^{60}(G(i) - \max(180, G_b))_+ \quad \text{(Equation 1)}$$

where $Q_b$ is the sum of the positive deviations for the control period of 60 cycles (i.e., 5 hours where each cycle is 5 minutes in length), $i$ is the cycle index within the control period, $G(i)$ is the glucose concentration of the user at cycle $i$, $G_b$ is the glucose concentration of the user at the time of delivery of the insulin bolus, and max is a maximum function that chooses a largest value among a set of values. The + in Equation 1 indicates that only positive deviations are summed. From Equation 1, it is evident that the sum of deviations is the sum of the differences for each cycle between the glucose concentration for the user at the cycle relative to the glucose concentration at the time of delivery of the insulin bolus.

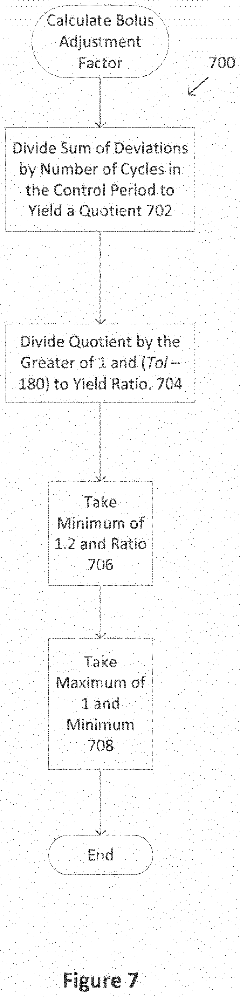

From the sum of the deviations $Q_b$, the bolus adjustment factor $b_f$ may be calculated at 604. Figure 7 depicts a flowchart 700 of illustrative steps that may be performed in exemplary embodiments to calculate $Q_b$. A suitable formula for calculating the bolus adjustment factor is:

$$b_f = \max\left(1, \min\left(1.2, \frac{Q_b}{60 \cdot \max(1, Tol - 180)}\right)\right) \quad \text{(Equation 2)},$$

where *Tol* is a tolerance deviation value that is the maximum acceptable value and min is a minimum function that selects a minimum among a set of values. *Tol* - 180, represents the difference between the expected deviation *Tol* and the hyperglycemic limit of 180 mg/dL. At 702, the sum of the positive deviations $Q_b$ is divided by the number of cycles in the control period 60, to get an average deviation per cycle. At 704, the resulting quotient is then divided by the greater of 1 and the difference between the expected deviation *Tol* and the hyperglycemic limit of 180 mg/dL. The resulting ratio is reflective of how much the average positive deviation cycle ($Q_b$/60) compares to the expected deviation over the hyperglycemic limit (*Tol*-180). At 706, the smaller of the ratio or 1.2 is chosen by the minimum function so as to limit the percentage change to 20% or less. Then, at 708, the maximum of 1 and the minimum function is chosen as the value of $b_f$. This ensures that $b_f$ is not less than one (which would decrease the insulin bolus dosage).

At 606, the insulin bolus dosage is adjusted by the bolus adjustment factor. Specifically, the insulin bolus dosage from the user is multiplied by the bolus adjustment factor. So, the adjusted insulin bolus dosage $b_a = b_r \times b_f$, where $b_r$ is the requested bolus dosage.

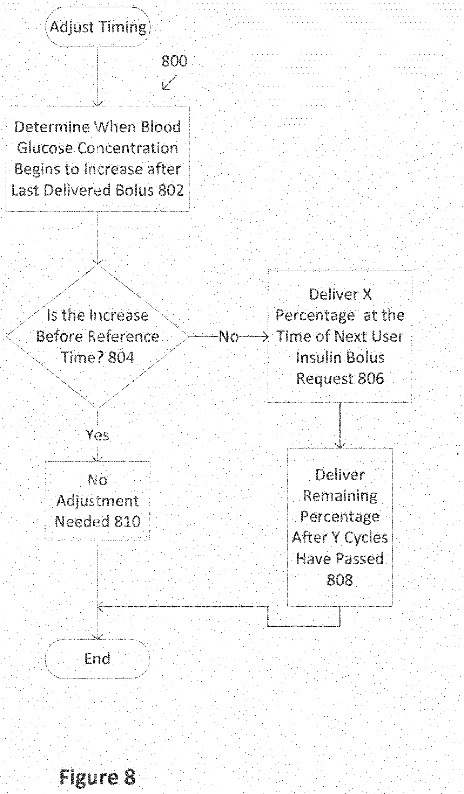

As was mentioned above, the timing of delivery of the insulin boluses may also be adjusted. Figure 8 depicts a flowchart 800 of illustrative steps that may be performed in exemplary embodiments to adjust the timing of delivery of the insulin boluses. At 802, the control application 116 or 120 examines glucose concentration values (such as on an ongoing basis or by examining data in the histories 111) for the user after the previous insulin bolus is delivered. The cycle at which the glucose concentration of the user begins to increase is identified. At 804, the time between when the insulin bolus was delivered and the when the glucose concentration began to increase is compared relative to a reference time (e.g., 30 minutes after delivery of the insulin bolus). If the increase occurs after the reference time, a timing adjustment is needed. For example, a percentage X of the insulin bolus dose may be delivered initially at 806 and a remaining Y percentage of the insulin bolus dose (Y=100%-X%) may be delivered at a later time at 808 (i.e., delayed a given number of cycles, such as 6 cycles). X and the delay amount are tunable parameters.

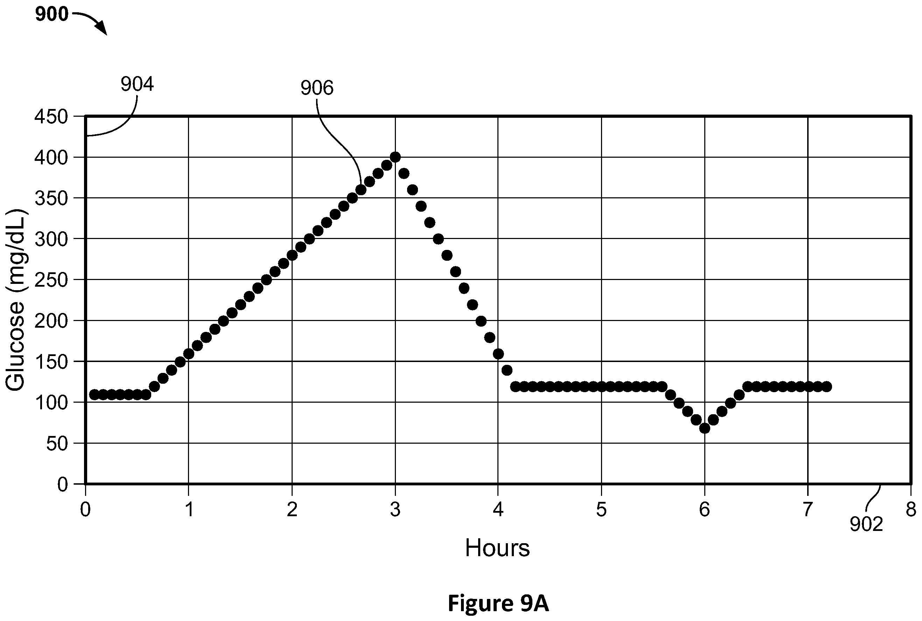

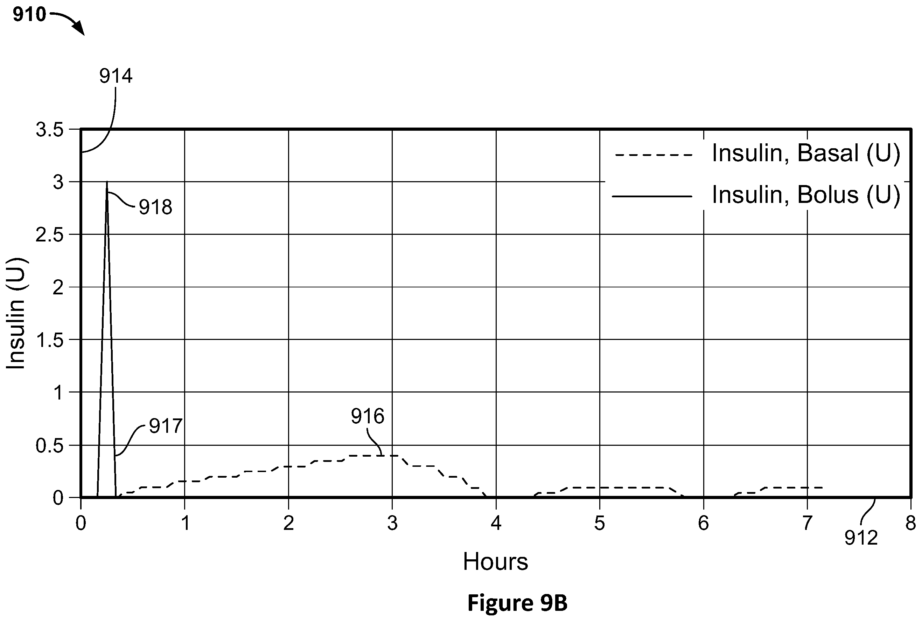

Figure 9A depicts a plot 900 of illustrative glucose concentration values for a user, and Figure 9B depicts a corresponding plot 910 of insulin deliveries for the user over a same time period. In Figure 9A, the X-axis 902 is time, and the Y-axis 904 is glucose concentration in mg/dL. For Figure 9B, the X-axis 912 is time and Y-axis 914 is insulin units. The glucose concentration values 906 in Figure 9A are plotted over a period slightly over seven hours, as are the insulin delivery values in Figure 9B.

Slightly after the 1-hour mark, the glucose concentration values 906 exceed the hyperglycemic threshold until the 3-hour mark. This is despite an insulin bolus delivery as indicated by peak 918 of curve 917. The basal insulin deliveries increase in response until the 3-hour mark as indicated curve 916, at which time the glucose concentration values 906 begin to decrease. The glucose concentration values decrease until slightly after the 4-hour mark and stay flat until about the 5.6-hour mark, where the glucose concentration values dip again briefly until the 6-hour mark. As can be seen in Figure 9B, the basal insulin 916 is halted before the 6-hour mark responsive to the decrease in glucose concentration values dipping. The halting causes an increase in glucose concentration until the basal insulin is reinitiated at a low dose.

In applying the method of Figure 8 to the situation represented by the plots of Figure 9A and 9B, the exemplary embodiments would calculate $Q_b$ to be equal to 533500, to account for glucose excursion above 180 between the 70th minute (1.16 hours) and 235th minute (3.91 hours) mark. Consequently, the resulting bolus adjustment factor would be calculated as $b_f$ = max(1, min(1.2, 533500/(60*(240-180)))) = 1.2. So, the control application 116 or 120 would recommend increasing the user's next bolus by 20% by using the 1.2 bolus adjustment factor. As to the timing of the delivery of the insulin dosage, the user delivered a 3U bolus at the 15-minute mark, and the glucose concentration rise began at the 40th minute mark, for a time difference of 25 minutes. Therefore, there would be no delay of the delivery of the next bolus, since less than 30 minutes had passed since the bolus delivery.

While exemplary embodiments have been described herein, various changes in form and detail relative to the exemplary embodiments may be made without departing from the intended scope of the appended claims.

**Claims**

1. A method performed by a processor (111, 119) for controlling a medicament delivery device (100), comprising:

gathering data regarding insulin delivery and glucose levels of a user (108);
analyzing with the processor the gathered data to identify factors indicating that an insulin to carbohydrate ratio for the user or the correction factor for the user should be increased; wherein the identified factors indicating that an insulin to carbohydrate ratio for the user or a correction factor for the user should be increased include at least one of:

the user being delivered less than a threshold number of insulin boluses in a time period, or
non-bolus insulin delivery to a user exceeds a percentage threshold;
analyzing with the processor the gathered data to identify factors indicating that the insulin to carbohydrate ratio for the user or the correction factor should be decreased, wherein the identified factors indicating that the insulin to carbohydrate ratio for the user or the correction factor for the user should be decreased include at least one of:

the user delivered more than a threshold number of insulin boluses in a time period;
bolus insulin delivery to the user exceeds a percentage threshold;
variability among glucose concentration readings for a user is greater than a variability threshold, or
the user activates a mode to protect against hypoglycemia more often than a frequency threshold;
weighing the identified factors with the processor to determine whether to increase or decrease the insulin to carbohydrate ratio or the correction factor; and
increasing or decreasing the insulin to carbohydrate ratio or the correction factor for the user based on the determination and the weighing of the identified factors to adjust insulin delivery by the medicament delivery device.

2. The method of one of the preceding claims, wherein weighing the identified factors comprises assigning weights to the identified factors.

3. The method of claim 2, wherein some of the weights are positive and some of the weights are negative.

4. The method of claim 2 or 3, comprising summing the weights to produce a final adjustment factor and comparing the final adjustment factor to one or more weight thresholds to decide whether to increase or decrease the insulin to carbohydrate ratio or the correction factor.

5. The method of one of the preceding claims, wherein increasing or decreasing the insulin to carbohydrate ratio or the correction factor comprises increasing both the insulin to carbohydrate ratio and the correction factor.

6. The method of one of the preceding claims, wherein increasing or decreasing the insulin to carbohydrate ratio or the correction factor as decided comprises decreasing both the insulin to carbohydrate ratio and the correction factor.

**Patentansprüche**

1. Verfahren, das von einem Prozessor (111, 119) zur Steuerung einer Arzneimittelabgabevorrichtung (100) durchgeführt wird, umfassend:

Sammeln von Daten über die Insulinabgabe und Glukosespiegel eines Benutzers (108);
mit dem Prozessor Analysieren der gesammelten Daten, um Faktoren zu identifizieren, die darauf hinweisen, dass ein Insulin-Kohlenhydrat-Verhältnis für den Benutzer oder der Korrekturfaktor für den Benutzer erhöht werden sollte; wobei die identifizierten Faktoren, die darauf hinweisen, dass ein Insulin-Kohlenhydrat-Verhältnis für den Benutzer oder ein Korrekturfaktor für den Benutzer erhöht werden sollte, mindestens einen der folgenden umfassen:

dem Anwender wird in einem Zeitraum weniger als eine Schwellenwertanzahl von Insulinboli verabreicht, oder
die Nicht-Bolus-Insulinabgabe an einen Benutzer überschreitet einen prozentualen Schwellenwert;

mit dem Prozessor Analysieren der gesammelten Daten, um Faktoren zu identifizieren, die darauf hinweisen, dass ein Insulin-Kohlenhydrat-Verhältnis für den Benutzer oder der Korrekturfaktor für den Benutzer verringert werden sollte, wobei die identifizierten Faktoren,
die darauf hinweisen, dass das Insulin-Kohlenhydrat-Verhältnis für den Benutzer oder der Korrekturfaktor für den Benutzer verringert werden sollte, mindestens einen der folgenden umfassen:

dem Anwender wird in einem Zeitraum mehr als eine Schwellenwertanzahl von Insulinboli verabreicht;
die Bolus-Insulinabgabe an den Benutzer überschreitet einen prozentualen Schwellenwert;
die Variabilität der Glukosekonzentrationswerte für einen Benutzer ist größer als eine Variabilitätsschwelle, oder
der Benutzer aktiviert einen Modus zum Schutz vor Hypoglykämie häufiger als eine Frequenzschwelle;
Gewichten der identifizierten Faktoren mit dem Prozessor, um zu bestimmen, ob das Insulin-Kohlenhydrat-Verhältnis oder der Korrekturfaktor erhöht oder verringert werden soll; und
Erhöhen oder Verringern des Insulin-Kohlenhydrat-Verhältnisses oder des Korrekturfaktors für den Benutzer basierend auf der Bestimmung und dem Gewichten der identifizierten Faktoren zur Anpassung der Insulinabgabe durch die Arzneimittelabgabevorrichtung.

2. Verfahren nach einem der vorhergehenden Ansprüche, wobei das Gewichten der identifizierten Faktoren das Zuweisen von Gewichten zu den identifizierten Faktoren umfasst.

3. Verfahren nach Anspruch 2, wobei einige der Gewichte positiv sind und einige der Gewichte negativ sind.

4. Verfahren nach Anspruch 2 oder 3, umfassend das Summieren der Gewichte, um einen endgültigen Anpassungsfaktor zu erzeugen, und das Vergleichen des endgültigen Anpassungsfaktors mit einer oder mehreren Gewichtsschwellen, um zu entscheiden, ob das Insulin-Kohlenhydrat-Verhältnis oder der Korrekturfaktor erhöht oder verringert werden soll.

5. Verfahren nach einem der vorhergehenden Ansprüche, wobei das Erhöhen oder Verringern des Insulin-Kohlenhydrat-Verhältnisses oder des Korrekturfaktors das Erhöhen sowohl des Kohlenhydratverhältnisses als auch des Korrekturfaktors umfasst.

6. Verfahren nach einem der vorhergehenden Ansprüche, wobei das Erhöhen oder Verringern des Insulin-Kohlenhydrat-Verhältnisses oder des Korrekturfaktors das Verringern sowohl des Kohlenhydratverhältnisses als auch des Korrekturfaktors umfasst.

**Revendications**

1. Procédé effectué par un processeur (111, 119) pour commander un dispositif d'administration de médicament (100), comprenant :

le rassemblement de données concernant l'administration d'insuline et des niveaux de glucose d'un utilisateur (108) ;
l'analyse, avec le processeur, des données rassemblées pour identifier des facteurs indiquant qu'un rapport insuline/glucides pour l'utilisateur ou le facteur de correction pour l'utilisateur doit être augmenté ; dans lequel les facteurs identifiés indiquant qu'un rapport insuline/glucides pour l'utilisateur ou un facteur de correction pour l'utilisateur doit être augmenté comportent au moins l'un des éléments suivants
l'utilisateur ayant reçu moins d'un nombre seuil de bolus d'insuline dans un laps de temps, ou l'administration d'insuline non-bolus à un utilisateur dépasse un pourcentage seuil ;
l'analyse, avec le processeur, des données rassemblées pour identifier des facteurs indiquant que le rapport insuline/glucides pour l'utilisateur ou le facteur de correction pour l'utilisateur doit être diminué, dans lequel les facteurs identifiés indiquant que le rapport insuline/glucides pour l'utilisateur ou le facteur de correction pour l'utilisateur doit être diminué comportent au moins l'un des éléments suivants :

l'utilisateur a reçu moins d'un nombre seuil de bolus d'insuline dans un laps de temps, ou
l'administration d'insuline bolus à un utilisateur dépasse un pourcentage seuil ;
la variabilité entre les relevés de concentration de glucose pour un utilisateur est plus grande qu'une variabilité seuil, ou

l'utilisateur active un mode de protection contre l'hypoglycémie plus souvent qu'une fréquence seuil ;

la pondération des facteurs identifiés avec le processeur pour déterminer s'il faut augmenter ou diminuer le rapport insuline/glucides ou le facteur de correction ; et

l'augmentation ou la diminution du rapport insuline/glucides ou du facteur de correction pour l'utilisateur en fonction de la détermination et de la pondération des facteurs identifiés pour ajuster l'administration d'insuline par le dispositif d'administration de médicament.

2. Procédé selon l'une des revendications précédentes, dans lequel la pondération des facteurs identifiés comprend l'attribution de pondérations aux facteurs identifiés.

3. Procédé selon la revendication 2, dans lequel certaines des pondérations sont positives et certaines des pondérations sont négatives.

4. Procédé selon la revendication 2 ou 3, comprenant la sommation des pondérations pour produire un facteur d'ajustement final et la comparaison du facteur d'ajustement final à une ou plusieurs pondérations seuils pour décider s'il faut augmenter ou diminuer le rapport insuline/glucides ou le facteur de correction.

5. Procédé selon l'une des revendications précédentes, dans lequel l'augmentation ou la diminution du rapport insuline/glucides ou du facteur de correction comprend l'augmentation à la fois du rapport de glucides et du facteur de correction.

6. Procédé selon l'une quelconque des revendications précédentes, dans lequel l'augmentation ou la diminution du rapport insuline/glucides ou du facteur de correction comme décidé comprend la diminution à la fois du rapport de glucides et du facteur de correction.

Figure 1

100

Storage 131
Data 129

Cloud Services/Server(s) 128

Network 124

Management Device 104
Processor 119
UI 123
Control App 120
Storage 118
Histories 121

Sensor(s) 106

User 108

Medicament Delivery Device 102
Controller 110
Reservoir 112
Pump 113
Histories 111
Storage 114
Control App. 116
User Interface 117

Computing Device 126

Network 122

Smartwatch 130

Fitness Monitor 132

Other Wearable Device 134

Figure 2

**300**

| Factor | Adjustment | Rationale |
|---|---|---|
| Hyperglycemia (glucose >180 mg/dL) at any point within 90 minutes of any of the user's boluses | 1 | This is likely caused by insulin under-delivery precipitated by the user's actions. |
| User delivered less than 3 boluses per day | 1 | The user is starting to overly rely on the insulin delivery of AID systems, and the boluses that are delivered by the user should be increased. |
| User experienced CGM > 300 mg/dL for more than 1 hour at any point in pump session | 1 | This is clinical definition for increased risk of ketosis. If this occurred despite AID, the user's bolus should be increased. |
| User's overall non-bolus insulin delivery is more than 60% of the user's total insulin needs | 1 | This also indicates that there is likely under-coverage of the user's insulin needs by boluses. |

302  304  306

308  310  312  314

**Figure 3A**

**300'**

| 302 | 304 | 306 |
|---|---|---|
| hypoglycemia (glucose < 70 mg/dL) at any point within 90 minutes of any of the user's boluses | -3 | This is likely caused by insulin over-delivery precipitated by the user's actions. |
| User delivered more than 8 boluses per day | -1 | The more times the user bolus, the more likely that the user may make a mistake in one of those boluses. |
| User experienced CGM < 54 mg/dL for any duration at any point in pump session | -1 | This is clinical definition for severe hypoglycemia |
| User's overall non-bolus insulin delivery is less than 40% of the user's total insulin needs | -1 | This indicates that user is likely over-bolusing for their insulin needs |
| User's CGM standard deviation is greater than 50 mg/dL | -1 | This indicates that the user's blood glucose is swinging wildly, likely due to the user's over-delivery followed by a compensating ingestion of fast-acting carbohydrates |
| User's overall activation of Hypo Protect Mode is more than 5% of the time | -1; must have any other negative factor to also account for this factor | This indicates that the user may be experiencing periods where their glucose control is not satisfactory, which may be significantly impacted by the user's manual bolus deliveries. |

**Figure 3B**

**330**

| Final Adjustment Factor | Resulting change in AID settings |
|---|---|
| <-2 | -10% |
| -2 to +3 | 0 |
| >3 | +10% |

332      334      336      338      340

**Figure 3C**

**Figure 4A**

**Figure 4B**

EP 4 141 879 B1

Figure 5

```
              ╭─────────────╮
              │   Adjust    │
              │   Dosage    │
              ╰──────┬──────╯          ╱ 600
                     │              ↙
                     ▼
    ┌────────────────────────────────┐
    │  Sum Deviations in Blood Glucose │
    │   Concentration (BGC) Between    │
    │   When Delivered Insulin Bolus   │
    │  and BGC at Each Cycle in Control│
    │           Period 602             │
    └────────────────┬─────────────────┘
                     │
                     ▼
          ┌──────────────────────┐
          │   Calculate Bolus    │
          │  Adjustment Factor   │
          │    Using Sum of      │
          │   Deviations 604     │
          └──────────┬───────────┘
                     │
                     ▼
          ┌──────────────────────┐
          │   For Next insulin   │
          │  Bolus, Mulitply Insulin│
          │   Dosage by Bolus    │
          │  Adjustment Factor   │
          │         606          │
          └──────────┬───────────┘
                     │
                     ▼
              ╭─────────────╮
              │     End     │
              ╰─────────────╯
```

## Figure 6

EP 4 141 879 B1

Calculate Bolus
Adjustment
Factor

700

Divide Sum of Deviations
by Number of Cycles in
the Control Period to
Yield a Quotient 702

Divide Quotient by the
Greater of 1 and (*Tol* −
180) to Yield Ratio. 704

Take
Minimum of
1.2 and Ratio
706

Take
Maximum of
1 and
Minimum
708

End

**Figure 7**

**Figure 8**

**Figure 9A**

**Figure 9B**

**EP 4 141 879 B1**